# EUROPEAN PATENT SPECIFICATION

(11) **EP 1 358 875 B1**
(45) Date of publication and mention of the grant of the patent: **11.01.2006**
(21) Application number: 03008724.1
(22) Date of filing: 16.04.2003
(51) Int. Cl.: A61K 9/48

(54) **Colored hard capsules**
Gefärbte Hartkapseln
Capsules dures colorées

(30) Priority: 30.04.2002 JP 2002127793
(43) Date of publication of application: 05.11.2003
(73) Proprietor: Warner-Lambert Company LLC, Morris Plains, New Jersey 07950 (US)
(72) Inventor: Taniguchi, Kazuyoshi, Capsugel Japan Inc., Sagamihara city, Kanagawa Pref. 229-1133 (JP); Ohnuki, Hiroshi, Capsugel Japan Inc., Sagamihara city, Kanagawa Pref. 229-1133 (JP); Sai, Eisaku, Capsugel Japan Inc., Sagamihara city, Kanagawa Pref. 229-1133 (JP); Miyata, Kenji, Capsugel Japan Inc., Sagamihara city, Kanagawa Pref. 229-1133 (JP)
(74) Representative: Tesch, Rudolf

(56) References cited:
- FR-A- 2 585 247
- US-A- 4 759 936
- US-A- 5 419 916
- PATENT ABSTRACTS OF JAPAN vol. 0185, no. 82, 8 November 1994 (1994-11-08) & JP 62 017718 A (KIKKOMAN CORP.)
- DATABASE WPI Week 199234 Derwent Publications Ltd., London, GB; AN 1992-281661 XP002245655 & JP 04 193825 A (NIPPON ELANCO KK), 13 July 1992 (1992-07-13)

## Description

### BACKGROUND OF THE INVENTION

The present invention relates to a hard capsule, more particularly, to a hard capsule having stable color comprising natural pigment or natural pigment derivative as a colorant.

Conventional coloring of hard capsules, in particular hard gelatin capsules, is mainly done for the purpose of ensuring identification in order to prevent them from being taken mistakenly or to produce pharmaceuticals conveniently. Enhancement of product value through aesthetic coloring is a secondary purpose. The coloring was almost always carried out through the use of synthetic tar pigment alone as stipulated in the Pharmaceutical Affairs Law, or by combining them with titanium dioxide, which is a white pigment complying with standards of the Japanese Pharmacopoeia. In addition, gelatin capsules using inorganic pigments like iron sesquioxide or yellow iron sesquioxide are also known.

In recent years, a demand for hard capsules has increased not only in the field of pharmaceuticals, but also in the field of foods focusing primarily on so-called health foods. However, there is still significant concern among ordinary consumers regarding synthetic colorants. In such a situation, from the viewpoint of appealing to consumers with greater safety, there is also a growing demand for hard capsules comprising natural pigment that is widely used as colorant in the field of foods in particular.

Several colored capsules that are suitably blended with natural pigment, instead of the above synthetic tar pigment or inorganic pigment, have already been reported. Example of such natural pigment includes β-carotene, caramel, sodium copper chlorophyllin, carminic acid, laccaic acid, carthamus yellow pigment or red cabbage pigment. Refer to, for example, Japanese Unexamined Patent Publication No. 54-138119, No. 55-161863 and No. 62-68860, Japanese Examined Patent Publication No. 60-43046 and Japanese Patent No. 2696271. FR-2585247 discloses hard capsules comprising natural pigments in the shell.

However, there are many cases in which natural pigments typically have inferior heat resistance, light resistance, oxidation-reduction resistance and pH stability as compared with synthetic pigments. Thus, even if the natural pigments can be widely used in the field of foods, a judgment as to whether or not pigments can be blended into a capsule cannot be made indiscriminately. Stability in the production process, including color change, pigment insolubilization/aggregation, pH change and viscosity change, and stability in the capsule after the production process, such as light resistance, are required in order to be blended into the capsule. In addition, the capsule blended with natural pigments also must satisfy the required properties for the capsule, such as dissolution behavior, film strength and so forth.

As a result of our studies for novel natural pigments capable of being blended into capsules, we found that one or more pigment(s) selected from the group consisting of gardenia red pigment, marigold pigment, purple corn pigment, tamarind pigment and sodium iron chlorophyllin, or the combination of titanium oxide and the above pigment(s) can be blended into a capsule base, such as gelatin, pullulan or HPMC, so that a satisfactory hard capsule that has aesthetic color and that satisfies the required stability and properties under practical conditions of production can be obtained.

### DETAILED DESCRIPTION OF THE INVENTION

The present invention relates to a hard capsule comprising from 0.01 to 5.0% by weight of natural pigment or natural pigment derivative, to be collectively referred to as natural pigment, selected from the group consisting of gardenia red pigment, marigold pigment, purple corn pigment, tamarind pigment and sodium iron chlorophyllin, or from 0.1 to 5.0% by weight of titanium dioxide together with from 0.01 to 5.0% by weight of the natural pigment.

Gardenia red pigment is a red pigment obtained by hydrolyzing a water or ethanol extract of gardenia fruits of the Rubiaceae (Gardenia augusta MERR. Var. grandiflora HORT.), followed by subjecting to the action of a food processing enzyme together with protein degradation product.

Marigold pigment is a yellow pigment obtained by extracting from marigold flowers of the Asteraceae (Tagetes erecta Willd.) with organic solvent, and has xanthophyll as its main component.

Purple corn pigment is a purple pigment obtained by extraction from purple seeds of corn of the Gramineae (Zea mays LINN), and has cyanidin-3-glucoside and so forth as its main components.

Tamarind pigment is a reddish-brown pigment obtained by extraction from tamarind seeds of the Leguminosae (Tamarindus indica LINNE), and has flavonoid as its main component.

Sodium iron chlorophyllin is a green pigment obtained by hydrolysis of iron chlorophyll, in which the magnesium in natural chlorophyll is replaced with iron, with dilute methanolic sodium hydroxide, and is classified as the natural pigment derivative.

The amount of these natural pigments is from 0.01 to 5.0% by weight relative to the hard capsule. In the case where each of these pigments is used alone, each preferable amount used from the point of dyeing effects is described as follows: the amount of gardenia red pigment, marigold pigment, purple corn pigment or tamarind pigment is from 0.1 to 3.0% by weight, and the amount of sodium iron chlorophyllin is from 0.02 to 0.75% by weight. In addition, the amount of titanium dioxide blended into the natural pigment is from 0.1 to 5.0% by weight relative to the hard capsule.

In using pigments or titanium dioxide, they should be added so as to be uniformly dispersed in an original solution of the capsule base during the production of the hard capsule. In addition, all of these pigments are added in the form of an aqueous solution. There is no particular need to define the order of addition. The object of the present invention can be adequately achieved when the colorant is added to the original solution of the capsule base in which titanium dioxide has been uniformly dispersed, or titanium dioxide can be dispersed in the original solution of the capsule base that has been colored in advance with a colorant solution.

Uniform dispersion does not require special machinery, but rather can be adequately carried out by stirring and dispersion using known apparatus and methods. With respect to capsule molding as well, the capsules having aesthetic color can be obtained by using a known capsule molding machine.

The hard capsule base may be a known base that can be used in pharmaceuticals and foods, examples of which include gelatin, pullulan, HPMC and the like. Preferred is gelatin capsules.

Moreover, the above pigments do not prohibit the use of plasticizers, preservatives, dispersants or other additives required in accordance with ordinary methods, and are able to adequately achieve the objective of coloring even in the presence of these additives.

Moreover, although the use of the above pigments enables molded capsules to respectively exhibit aesthetic colors according to each pigment, namely red color by gardenia red pigment, yellow color by marigold pigment, purple color by purple corn pigment, reddish-brown color by tamarind pigment and green color by sodium iron chlorophyllin pigment, these colors are stable to both the passage of time and light. In addition, the color of the capsule can be continuously varied according to the amounts of pigment and titanium dioxide used, and can be adjusted to a fixed color by maintaining a fixed blended amount.

The capsules colored by the pigments of the present invention are stable to the passage of time and light. For example, gelatin capsules of the present invention are suitable to capsule quality, namely appearance, physical properties and purity tests stipulated by the Japanese Pharmacopoeia. And furthermore when their ability of dissolution is tested in accordance with the dissolution test defined in the general test and assay of the Japanese Pharmacopoeia, the gelatin capsules of the present invention exhibit a dissolution behavior completely similar to conventional capsules. Therefore they can be used for pharmaceuticals or foods.

The following examples provide a detailed explanation of the present invention

### EXAMPLES

### Example 1

To confirm the stability in the production process (color change, pigment insolubilization/aggregation), 1.0% by weight of various natural pigments (0.1% by weight of sodium iron chlorophyllin so as to match the color of other pigments) were added to a 30% gelatin solution followed by storing for 48 hours at 50°C. Those results are shown in Table 1. Pigment insolubilization and aggregation were not observed. On the other hand, as shown in Table 1, considerable discoloration in the gelatin solution was observed for monascus pigment, gardenia yellow pigment and purple sweet potato pigment.

**Table 1**

| Pigment | Amt. Added (% by weight) *1 | Color change |
|---|---|---|
| Gardenia red pigment | 1.0% | No change |
| Monascus pigment | 1.0% | Discoloration |
| Gardenia yellow pigment | 1.0% | Discoloration |
| Marigold pigment | 1.0% | No change |
| Riboflavin | 1.0% | No change |
| Purple corn pigment | 1.0% | No change |
| Purple sweet potato pigment | 1.0% | Discoloration |
| Tamarind pigment | 1.0% | No change |
| Sodium iron chlorophyllin | 0.1% | No change |

| | | |
|---|---|---|
| *1: Amount added relative to gelatin powder | | |

### Example 2

An aqueous solution of gardenia red pigment was added to a gelatin solution for which viscosity had been adjusted followed by uniformly mixing. The amount of gardenia red pigment added to the gelatin solution is as shown in Table 2. Hard gelatin capsules were then molded from this mixture in accordance with ordinary methods. The resulting capsules were transparent, and exhibited a satisfactory red tinted color. As is clear from Table 2, the capsules colored with gardenia red pigment were confirmed to be completely free of problems with stability to both the passage of time and light.

**Table 2**

| Pigment | Amt. Added (% by weight)*1 | 5 months at room temp. | 3 month's at 40°C and 75% RH | 120 hours under a white fluorescent lamp at 700 lux |
|---|---|---|---|---|
| Gardenia red pigment | 1.0% | No change | No change | Equivalent to conventional capsules*2 |

| | | | | |
|---|---|---|---|---|
| *1: Amount added to gelatin solution | | | | |
| *2: Compared with control capsules colored with conventional tar-based pigment (light red and light blue) | | | | |

### Examples 3-7

Hard gelatin capsules were molded in the same manner as Example 2 using aqueous solutions of the natural pigments shown in the following table instead of gardenia red pigment. The amounts of natural pigments added to the gelatin solution are as shown in Table 3. The resulting capsules were transparent and exhibited satisfactorily aesthetic colors in the form of yellow tint in the case of marigold pigment and riboflavin, purple tint in the case of purple corn pigment, reddish-brown tint in the case of tamarind pigment and green tint in the case of sodium iron chlorophyllin. As is shown in Table 3, the capsules containing riboflavin exhibited discoloration due to light. On the other hand, the capsules colored with the other natural pigments were confirmed to be completely free of problems with respect to stability to both the passage of time and light.

**Table 3**

| Ex. No. | Pigment | Amt. Added (% by weight)*1 | 5 months at room temperature | 3 months at 40°C and 75% RH | 120 hours under a white fluorescent lamp at 700 lux |
|---|---|---|---|---|---|
| 3 | Marigold pigment | 1.0% | No change | No change | Equivalent to conventional capsules |
| 4 | Riboflavin | 1.0% | No change | No change | Discoloration |
| 5 | Purple corn pigment | 1.0% | No change | No change | Equivalent to conventional capsules |
| 6 | Tamarind pigment | 1.0% | No change | No change | Equivalent to conventional capsules |
| 7 | Sodium iron chlorophyllin | 0.1% | No change | No change | Equivalent to conventional capsules |

| | | | | | |
|---|---|---|---|---|---|
| *1,*2: Same meanings as Table 2 | | | | | |

### Example 8

Capsules colored with gardenia red pigment were molded in the same manner as Example 2 after titanium dioxide was blended uniformly. The amounts of gardenia red pigment and titanium dioxide added to the gelatin solution are as shown in Table 4. The resulting capsules were opaque and exhibited a satisfactory red tinted color. As is clear from Table 4, the capsules colored with gardenia red pigment and titanium dioxide were confirmed to be completely free of problems with respect to stability to both the passage of time and light.

**Table 4**

| | Amt. Added (% by weight)*1 | Titanium dioxide*1 (% by weight) | 5 months at room temperature | 3 months at 40°C and 75% RH | 120 hours under a white fluorescent lamp at 700 lux |
|---|---|---|---|---|---|
| Gardenia red pigment | 1.0% | 0.9% | No change | No change | Equivalent to conventional capsules |

| | | | | | |
|---|---|---|---|---|---|
| *1,*2: Same meanings as Table 2 | | | | | |

### Examples 9-12

Capsules colored by natural pigments were molded in the same manner as Example 2 after titanium dioxide was blended uniformly. The amounts of the natural pigments and titanium dioxide added to the gelatin solution are as shown in Table 5. The resulting capsules were opaque and exhibited satisfactorily aesthetic colors in the form of yellow tint in the case of marigold, purple tint in the case of purple corn pigment, reddish-brown tint in the case of tamarind pigment and green tint in the case of sodium iron chlorophyllin. As is clear from Table 5, the capsules colored with these natural pigments and titanium dioxide were confirmed to be completely free of problems with respect to stability to both the passage of time and light.

**Table 5**

| Ex. No. | Pigment | Amt. Added*1 (% by weight) | Titanium dioxide*1 (% by weight) | 5 months at room temp. | 3 months at 40°C and 75% RH | 120 hours under a white fluorescent lamp at 700 lux |
|---|---|---|---|---|---|---|
| 9 | Marigold pigment | 1.0% | 0.9% | No change | No change | Equivalent to conventional capsules |
| 10 | Purple corn pigment | 1.0% | 0.9% | No change | No change | Equivalent to conventional capsules |
| 11 | Tamarind pigment | 1.0% | 0.9% | No change | No change | Equivalent to conventional capsules |
| 12 | Sodium iron chlorophyllin | 0.1% | 0.9% | No change | No change | Equivalent to conventional capsules |

| | | | | | | |
|---|---|---|---|---|---|---|
| *1,*2: Same meanings as Table 2 | | | | | | |

### Example 13

A solution test for capsules obtained in above-mentioned Examples 2, 3 and 5-12 using the natural pigments was carried out using purified water warmed to 37°C±1°C under the standard conditions stipulated in the Japanese Pharmacopoeia, while conventional uncolored transparent and white opaque gelatin capsules were used as the control. The results obtained are shown in Table 6. As is clear from these results as well, there were hardly any differences in the solution time between the capsules of the present invention and the conventional capsules.

**Table 6**

| | Amt. of pigment added*1 (% by weight) | Titanium dioxide*1 (% by weight) | Solution time (min.) | | |
|---|---|---|---|---|---|
| | | | Mean | Min. | Max. |
| Gardenia red pigment | 1.0% | 0% | 4.0 | 4 | 4 |
| | | 0.9% | 3.2 | 3 | 4 |
| Marigold pigment | 1.0% | 0% | 4.4 | 4 | 5 |
| | | 0.9% | 4.4 | 4 | 5 |
| Purple corn pigment | 1.0% | 0% | 4.2 | 4 | 5 |
| | | 0.9% | 4.4 | 4 | 5 |
| Tamarind pigment | 1.0% | 0% | 4.6 | 4 | 5 |
| | | 0.9% | 3.8 | 3 | 4 |
| Sodium iron chlorophyllin | 0.1% | 0% | 4.2 | 4 | 5 |
| | | 0.9% | 4.0 | 3 | 5 |
| (Control) Colorless, transparent | - - | 0% | 4.4 | 4 | 5 |
| (Control) White, opaque | - - | 0.9% | 3.8 | 3 | 5 |

| | | | | | |
|---|---|---|---|---|---|
| *1: Same meaning as Table 2 | | | | | |

As has been described above, according to the present invention, by using the natural pigments that have been widely used in the past as food colorants, in particular gardenia red pigment, marigold pigment, purple corn pigment, tamarind pigment and sodium iron chlorophyllin, the hard capsules that have aesthetic color and luster can be provided.

The capsules of the present invention can be used for pharmaceuticals or foods and are extremely useful, and in gelatin capsules, for example, be suitable to capsule quality, namely appearance, physical properties and purity tests stipulated by the Japanese Pharmacopoeia. Moreover, their visual quality has also been confirmed to be stable with respect to both the passage of time and light.

## Claims

1. A hard capsule comprising from 0.01 to 5.0% by weight of a pigment selected from the group consisting of gardenia red pigment, marigold pigment, purple corn pigment, tamarind pigment, sodium iron chlorophyllin and mixtures thereof.

2. The hard capsule according to claim 1, further comprising from 0.1 to 5.0% by weight of titanium dioxide.

3. The hard capsule according to claim 1, wherein said pigment is selected from the group consisting of gardenia red pigment, marigold pigment, purple corn pigment or tamarind pigment from 0.1 to 3.0% by weight.

4. The hard capsule according to claim 1, wherein the pigment is sodium iron chlorophyllin from 0.02 to 0.75% by weight.

5. The hard capsule according to claim 3, further comprising from 0.1 to 5.0% by weight of titanium dioxide.

6. The hard capsule according to claim 4, further comprising from 0.1 to 5.0% by weight of titanium dioxide.

## Patentansprüche

1. Hartkapsel, die 0,01 bis 5,0 Gew.-% eines Pigments umfasst, das aus der Gruppe Rotpigment der Gardenie, Pigment der Ringelblume, Purpurmaispigment (purple corn pigment), Pigment der Tamarinde, Natroneisenchlorphyllin und Gemischen hiervon ausgewählt ist.

2. Hartkapsel nach Anspruch 1, die ferner 0,1 bis 5,0 Gew.-% Titandioxid umfasst.

3. Hartkapsel nach Anspruch 1, wobei das Pigment aus der Gruppe Rotpigment der Gardenie, Pigment der Ringelblume, Purpurmaispigment oder Pigment der Tamarinde in einer Menge von 0,1 bis 3,0 Gew.-% ausgewählt ist.

4. Hartkapsel nach Anspruch 1, wobei das Pigment Natroneisenchlorophyllin in einer Menge von 0,02 bis 0,75 Gew.-% ist.

5. Hartkapsel nach Anspruch 3, die ferner 0,1 bis 5,0 Gew.-% Titandioxid umfasst.

6. Hartkapsel nach Anspruch 4, die ferner 0,1 bis 5,0 Gew.-% Titandioxid umfasst.

## Revendications

1. Capsule dure comprenant 0,01 à 5,0% en poids d'un pigment choisi dans le groupe constitué par le pigment de gardénia rouge, le pigment de tagète, le pigment de maïs pourpre, le pigment de tamarin, le sel sodique de la chlorophylline ferrique et les mélanges de ceux-ci.

2. Capsule dure selon la revendication 1, comprenant en outre 0,1 à 5,0% en poids de dioxyde de titane.

3. Capsule dure selon la revendication 1, dans laquelle ledit pigment, choisi dans le groupe constitué par le pigment de gardénia rouge, le pigment de tagète, le pigment de maïs pourpre ou le pigment de tamarin, est contenu à raison de 0,1 à 3,0% en poids.

4. Capsule dure selon la revendication 1, dans laquelle le pigment, le sel sodique de la chlorophylline ferrique, est contenu à raison de 0,02 à 0,75% en poids.

5. Capsule dure selon la revendication 3, comprenant en outre 0,1 à 5,0% en poids de dioxyde de titane.

6. Capsule dure selon la revendication 4, comprenant en outre 0,1 à 5,0% en poids de dioxyde de titane.
